# EUROPEAN PATENT APPLICATION

(11) **EP 4 063 478 A1**
(43) Date of publication of application: **28.09.2022**
(21) Application number: 20908148.8
(22) Date of filing: 21.12.2020
(51) Int. Cl.: C12M 1/00, C12M 3/00

(54) **ADHERENT CELL CULTURE VESSEL, AND METHOD FOR PRODUCING ADHERENT CELL CULTURE VESSEL**

(30) Priority: 26.12.2019 JP 2019237046
(71) Applicant: Toyo Seikan Group Holdings, Ltd., Shinagawa-ku Tokyo 141-8627 (JP)
(72) Inventor: MATSUOKA Yosuke, Yokohama-shi, Kanagawa 240-0062 (JP); TANAKA Satoshi, Yokohama-shi, Kanagawa 240-0062 (JP); KOSEKI Osamu, Yokohama-shi, Kanagawa 240-0062 (JP); TOTANI Takahiko, Yokohama-shi, Kanagawa 240-0062 (JP); NISHIYAMA Takaharu, Yokohama-shi, Kanagawa 240-0062 (JP)
(74) Representative: Hasegawa, Kan
(86) International application number: PCT/JP2020/047599
(87) International publication number: WO 2021/132121

(57) **Abstract**

Provided is a culture vessel for adherent cells that has a large culture portion surface area and ensures easily dissociating and recovering cells without consuming a medium or a dissociation solution more than necessary. The bag-shaped adherent cell culture vessel made of flexible packaging material includes a vessel body portion (1) having a first vessel wall (11) and a second vessel wall (12) and one or more injecting/ejecting ports (2). The vessel body portion (1) has an intermediate culture wall (13) between the first vessel wall (11) and the second vessel wall (12). A culture chamber is disposed in each of between the first vessel wall (11) and the intermediate culture wall (13) and between the second vessel wall (12) and the intermediate culture wall (13), and a flow passage (14) communicating between the respective culture chambers is disposed.

## Description

### TECHNICAL FIELD

The present invention relates to a cell culture technique and particularly to a culture vessel for adherent cells.

### BACKGROUND ART

Recently, in the production of pharmaceutical medicines and in the fields of gene therapy, regeneration medicine, immunotherapy, and the like, it is desired that cells and tissues are efficiently cultured in a large amount under an artificial environment.

When adherent cells, such as induced pluripotent stem cells (iPS cells), neural stem cells, embryonic stem cells (ES cells), mesenchymal stem cells, hepatic cells, islet cells, cardiac muscle cells, corneal endothelial cells, and lymph corpuscles in an activation process, are made to adhere to a surface (culture portion) for culturing cells inside a culture vessel and cultured in a large amount, its yield is constrained by the surface area of the culture portion. In view of this, it is beneficial if the yield of the adherent cells can be increased by producing a culture vessel having a large surface area of the culture portion.

As the culture vessel having a large surface area of the culture portion, for example, multi-stage flasks in which a plurality of culture portions are stacked in a multi-stage manner to form a culture vessel and enable passing a medium between the culture portions are commercially available (such as, CellSTACK, which is a registered trademark of Corning Incorporated, Cell Factory System, which is a registered trademark of Thermo Fisher Scientific Inc., and CellCube of Astec Co., Ltd.). Using the multi-stage culture vessel allows culturing the adherent cells in a large amount.

Patent Document 1: WO 2016/190312 brochure

### SUMMARY OF THE INVENTION

### PROBLEMS TO BE SOLVED BY THE INVENTION

However, when the multi-stage culture vessel is used, there is a problem that a culture medium and a dissociation solution are consumed more than necessary.

That is, in order to effectively utilize the culture portions using the vessel, when adherent cells are made to adhere to the upper surface and the lower surface of each stage in the vessel and cultured (hereinafter, a method of culturing using both the upper surfaces and the lower surfaces in the vessel is referred to as a double-sided culture in some cases), the medium should be filled up to the brim in the vessel. However, when the double-sided culture is conducted using the rigid (hard) vessel having a constant inner volume, an originally required amount or more of the medium should be used even at the beginning of the culture when the number of cells is small.

Further, since the dissociation solution should be filled up to the brim in the vessel to cause the dissociation solution to act on the cells when the cells are recovered, there is a problem that a large excess amount is consumed compared with an ordinary protocol.

That is, since the adherent cells adhere to the culture portions as well as the cells adhere to one another, the adherent cells directly cannot be stripped from the culture portions with ease and recovered even by, for example, beating the culture vessel. Therefore, although, generally, dissociation of cells is performed using the dissociation solution (such as cell dissociation enzyme, TrypLE^{™} Select, which is a registered trademark of Thermo Fisher Scientific Inc.), there is a problem that the dissociation solution is consumed more than necessary.

Furthermore, when cells having a strong adhesiveness to a vessel is cultured, it is necessary to scrape the cells with a scraper after causing the dissociation solution to act or to apply a solution to the cells with a pipette to strip the cells.

However, when the multi-stage culture vessel is used, it is difficult to perform pipetting on the culture portions in each stage, and even when a shock, such as beating from the outside of the vessel, is applied after the dissociation solution is used, the cells cannot be sufficiently dissociated, which causes a problem of decrease in a cell recovery rate.

Here, Patent Document 1 discloses that a double-sided culture is conducted by making the adherent cells to adhere to the upper surface and the lower surface in a culture bag. However, there is a problem that in the culture bag, the surface area of the culture portions significantly reduces compared with the multi-stage culture vessel.

Therefore, the inventors have seriously studied and succeeded in developing an adherent cell culture vessel that has a large culture portion surface area and ensures easily dissociating and recovering cells without consuming a medium or a dissociation solution more than necessary by ensuring forming four or more surfaces of culture portions in a bag-shaped culture vessel made of flexible packaging material.

That is, an object of the present invention is to provide an adherent cell culture vessel that has a large culture portion surface area and ensures easily dissociating and recovering cells without consuming a medium or a dissociation solution more than necessary and a method for producing the adherent cell culture vessel.

### SOLUTIONS TO THE PROBLEMS

In order to achieve the above object, an adherent cell culture vessel of the present invention is a bag-shaped adherent cell culture vessel made of flexible packaging material that includes a vessel body portion having a first vessel wall and a second vessel wall, and one or more injecting/ejecting ports. The vessel body portion has an intermediate culture wall between the first vessel wall and the second vessel wall. A culture chamber is disposed in each of between the first vessel wall and the intermediate culture wall and between the second vessel wall and the intermediate culture wall, and a flow passage communicating between the respective culture chambers is disposed.

A method for producing adherent cell culture vessel of the present invention is a method for producing a bag-shaped adherent cell culture vessel made of flexible packaging material that includes: forming a plurality of grooves composed of mountain-shaped portions and trough-shaped portions on three or more flexible packaging material films; arranging a surface of the flexible packaging material films on which the grooves are formed in the culture vessel; heat-sealing a peripheral edge portion of the flexible packaging material films with one or more injecting/ejecting ports interposed between the flexible packaging material films; and performing vacuum forming, pressure forming, or vacuum pressure forming to form a culture chamber between the flexible packaging material films and a flow passage communicating between the respective culture chambers.

A method for producing adherent cell culture vessel of the present invention is a method for producing a bag-shaped adherent cell culture vessel made of flexible packaging material that includes: forming a bulging shape that bulges outward in a peripheral edge region of two flexible packaging material films by vacuum forming, pressure forming, or vacuum pressure forming to form a first vessel wall and a second vessel wall; forming a bulging shape that bulges outward in a peripheral edge region of flexible packaging material films used other than for an intermediate culture wall arranged in a center, when a plurality of intermediate culture walls are disposed in the adherent cell culture vessel, by vacuum forming, pressure forming, or vacuum pressure forming to form respective intermediate culture walls; and heat-sealing a peripheral edge portion of the flexible packaging material films with one or more injecting/ejecting ports and the intermediate culture walls interposed between the first vessel wall and the second vessel wall to form a culture chamber between the flexible packaging material films and a flow passage communicating between the respective culture chambers.

### EFFECTS OF THE INVENTION

With the present invention, an adherent cell culture vessel that has a large culture portion surface area and ensures easily dissociating and recovering cells without consuming a medium or a dissociation solution more than necessary and a method for producing the adherent cell culture vessel can be provided.

### BRIEF DESCRIPTION OF THE DRAWINGS

Fig. 1 is a drawing illustrating a plan view and a cross-sectional view of an adherent cell culture vessel according to an embodiment of the present invention.
Fig. 2 is a drawing illustrating configurations of a first vessel wall, a second vessel wall, and an intermediate culture wall in the adherent cell culture vessel according to the embodiment of the present invention.
Fig. 3 is a drawing illustrating configurations of a modification of the first vessel wall, the second vessel wall, and the intermediate culture wall in the adherent cell culture vessel according to the embodiment of the present invention.
Fig. 4 is a drawing illustrating configurations of another modification of the first vessel wall, the second vessel wall, and the intermediate culture wall in the adherent cell culture vessel according to the embodiment of the present invention.
Fig. 5A is a drawing illustrating a cross-sectional view of an application example of the adherent cell culture vessel according to the embodiment of the present invention.
Fig. 5B is a drawing illustrating a cross-sectional view of an application example of the adherent cell culture vessel according to the embodiment of the present invention.
Fig. 6 is a drawing illustrating photographs showing a perspective view and a right-side view of the adherent cell culture vessel according to the embodiment of the present invention.
Fig. 7 is a cross-sectional explanatory view illustrating a configuration and the like of a groove that can be formed in a culture portion in the adherent cell culture vessel according to the embodiment of the present invention.
Fig. 8 is a drawing illustrating photomicrographs showing V-shaped grooves formed on the culture portion in the adherent cell culture vessel according to the embodiment of the present invention and a cross section perpendicular to a direction in which the V-shaped grooves extend.

### DESCRIPTION OF PREFERRED EMBODIMENTS

The following describes an embodiment of an adherent cell culture vessel and a method for producing the adherent cell culture vessel of the present invention in detail with reference to the drawings. However, the present invention is not limited to a specific content of the following embodiment.

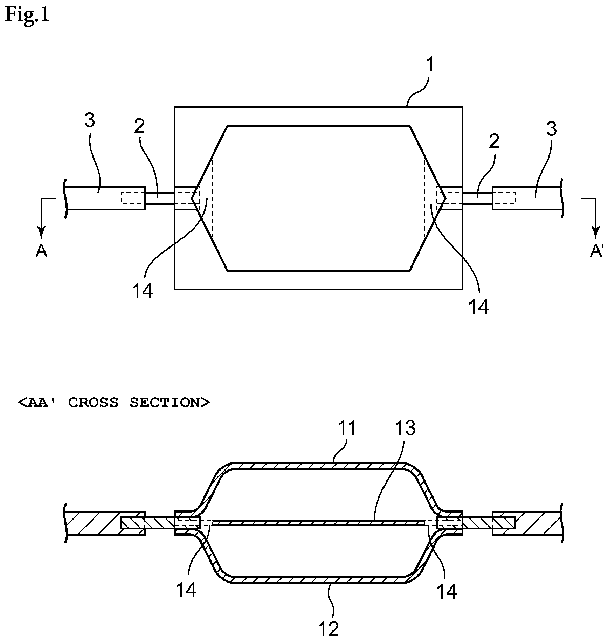

The adherent cell culture vessel of this embodiment is, for example, as illustrated in Fig. 1, a bag-shaped adherent cell culture vessel made of flexible packaging material and includes a vessel body portion 1 having a first vessel wall 11 and a second vessel wall 12 and one or more injecting/ejecting ports 2. The vessel body portion 1 has an intermediate culture wall 13 between the first vessel wall 11 and the second vessel wall 12, a culture chamber is each disposed between the first vessel wall 11 and the intermediate culture wall 13 and between the second vessel wall 12 and the intermediate culture wall 13, and flow passages 14 communicating between the respective culture chambers are disposed.

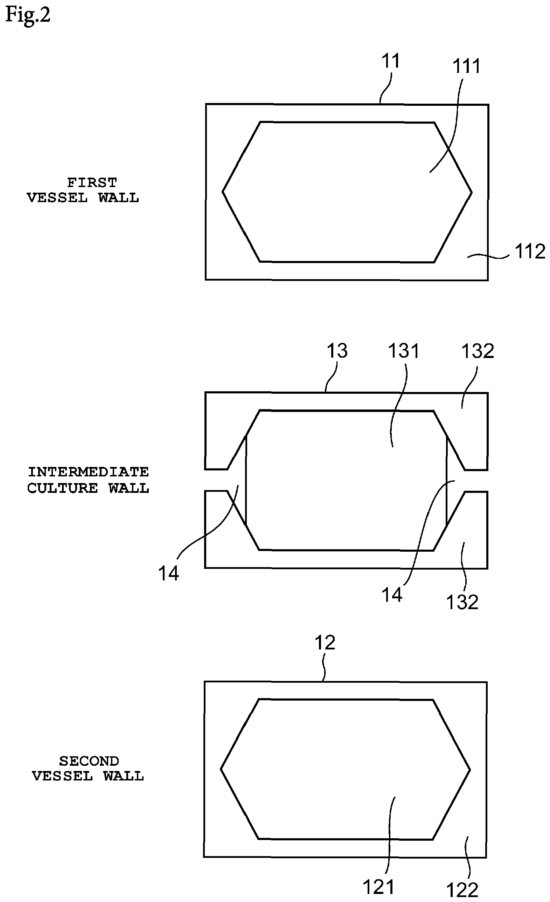

As illustrated in Fig. 2, as the first vessel wall 11, a rectangular film can be used. One surface of the film constitutes an outer surface of the adherent cell culture vessel, and the other surface constitutes an inner surface of the adherent cell culture vessel. The inner surface is composed of a culture portion 111 that is a region (culture surface) where adherent cells are made to adhere and cultured and a seal portion 112 that is a region on which the film is heat-sealed (thermally welded) in the peripheral area of the culture portion 111.

As the second vessel wall 12, a rectangular film can be also used. One surface of the film constitutes an outer surface of the adherent cell culture vessel, and the other surface constitutes an inner surface of the adherent cell culture vessel. Similarly to the first vessel wall 11, the inner surface is composed of a culture portion 121 that is a region where the adherent cells are made to adhere and cultured and a seal portion 122 that is a region on which the film is heat-sealed in the peripheral area of the culture portion 121.

The intermediate culture wall 13 is an intermediate layer disposed for increasing a culture area, and an approximately rectangular film can be used. The intermediate culture wall 13 is interposed between the first vessel wall 11 and the second vessel wall 12, and a part of both surfaces of the film are configured as culture portions in the adherent cell culture vessel.

That is, both surfaces of the intermediate culture wall 13 are composed of culture portions 131 that are regions where the adherent cells are made to adhere and cultured and seal portions 132 that are regions on which the film is heat-sealed in the peripheral areas of the culture portions 131.

The culture portions 131 in the intermediate culture wall 13 have cutouts (or holes passing through the intermediate culture wall) formed in parts of the regions corresponding to the culture portion 111 of the first vessel wall 11 and the culture portion 121 of the second vessel wall 12. The cutouts constitute the flow passages 14 communicating between the culture chamber formed between the first vessel wall 11 and the intermediate culture wall 13 and the culture chamber formed between the second vessel wall 12 and the intermediate culture wall 13.

When the adherent cell culture vessel is thus configured, as the culture portions in the adherent cell culture vessel, a total of four surfaces of the culture portion 111, the culture portion 121, and the two culture portions 131 can be used. Therefore, compared with a case where a double-sided culture is conducted using a culture vessel that does not include the intermediate culture wall 13, the culture area can be approximately doubled.

Since the adherent cell culture vessel of this embodiment is a bag-shaped vessel made of flexible packaging material, the capacity in the vessel can be controlled depending on the amounts of a medium and a dissociation solution injected into the vessel. In view of this, the medium and the dissociation solution are not consumed more than necessary.

Furthermore, by applying a force from the outside of the adherent cell culture vessel or by bringing the culture portions into contact with one another after the dissociation solution is made to act, dissociation and recovery of the cells can be easily performed.

As a material of the films constituting the first vessel wall 11, the second vessel wall 12, and the intermediate culture wall 13 in the adherent cell culture vessel of this embodiment, a polyolefin-based resin, such as polyethylene and polypropylene, and the like can be preferably used. For example, polyethylene, a copolymer of ethylene and α-olefin, a copolymer of ethylene and vinyl acetate, an ionomer using: a copolymer of ethylene and an acrylic acid or ethylene and a methacrylic acid; and a metal ion, and the like can be listed. Further, a polyolefin, a styrene-based elastomer, a polyester-based thermoplastic elastomer, a silicone-based thermoplastic elastomer, a silicone resin, and the like can be used. Furthermore, a silicone rubber, a soft vinyl chloride resin, a polybutadiene resin, an ethylene-vinyl acetate copolymer, a chlorinated polyethylene resin, a polyurethane-based thermoplastic elastomer, a polyester-based thermoplastic elastomer, a silicone-based thermoplastic elastomer, a styrene-based elastomer, such as styrene-butadiene-styrene (SBS), styrene-isoprene-styrene (SIS), styrene-ethylene-butylene-styrene (SEBS), and styrene-ethylene-propylene-styrene (SEPS), a polyolefin resin, a fluorine-based resin, and the like may be used.

The port 2 is to inject or eject (inject or discharge) the medium and the dissociation solution into or out of the adherent cell culture vessel, and the two ports 2 are disposed in the adherent cell culture vessel in Fig. 1. However, the number of the ports disposed in the adherent cell culture vessel is not limited to two pieces and may be one piece or three or more pieces. A tube 3 is coupled to the port 2. The tube 3 can be appropriately coupled to a medium supply vessel, a cell recovery vessel, an enzyme supply vessel, a disposal vessel, and the like that are not illustrated.

As a material of the port 2, for example, polyethylene, polypropylene, vinyl chloride, a polystyrene-based elastomer, a thermoplastic resin, such as FEP, and the like can be used.

As a material of the tube 3, for example, a silicone resin, a soft vinyl chloride resin, a polybutadiene resin, an ethylene-vinyl acetate copolymer, a polyurethane-based thermoplastic elastomer, a polyester-based thermoplastic elastomer, a silicone-based thermoplastic elastomer, a styrene-based elastomer, such as styrene-butadiene-styrene (SBS), styrene-isoprene-styrene (SIS), styrene-ethylene-butylene-styrene (SEBS), and styrene-ethylene-propylene-styrene (SEPS), a polyolefin resin, a fluorine-based resin, and the like can be used.

In Fig. 2, while the rectangular or the approximately rectangular film is used as the first vessel wall 11, the second vessel wall 12, and the intermediate culture wall 13, the first vessel wall 11, the second vessel wall 12, and the intermediate culture wall 13 are not limited to these. Films or sheets having other shapes, such as circle, oval, and polygon, can be used. While the shape of the culture portion 111 is hexagonal, the shape is not limited to this and may be rectangular, circular, oval, and the like.

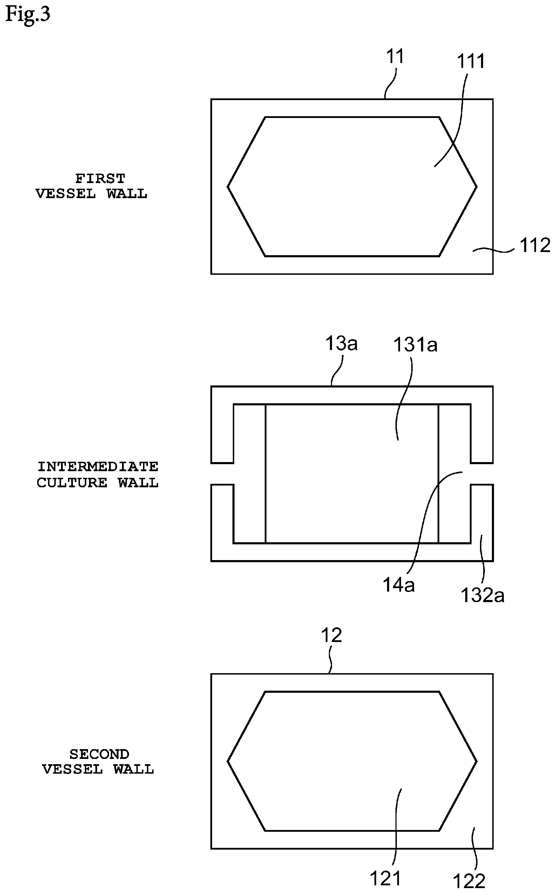

While the flow passages 14 formed in the intermediate culture wall 13 are formed in an approximately triangular shape at both ends of the culture portion 121 in Fig. 2, the flow passages 14 are not limited to this and can be, for example, approximately oblong in shape as illustrated by flow passages 14a illustrated in an intermediate culture wall 13a of Fig. 3.

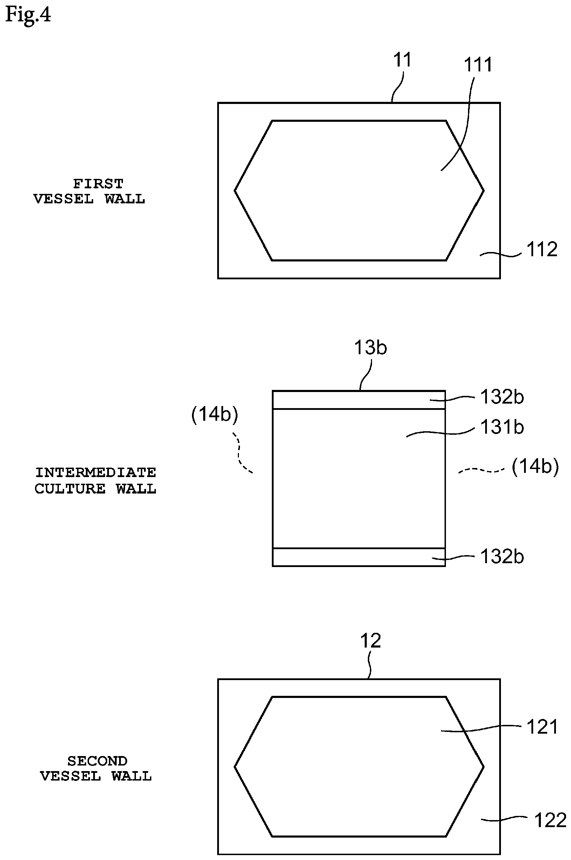

Furthermore, as illustrated in Fig. 4, flow passages 14b can be formed in the vicinity of ports on both sides of a culture portion 131b by using a film shorter than the first vessel wall 11 and the second vessel wall 12 as an intermediate culture wall 13b and performing heat-sealing with the film interposed between the first vessel wall 11 and the second vessel wall 12.

In Fig. 1 to Fig. 4, while the two flow passages 14 (14a, 14b) are disposed in the adherent cell culture vessel, the configuration is not limited to this. The configuration can be such that only any one of them is disposed or three or more are disposed. The positions of the flow passages 14 are not limited to these, and the flow passages 14 can be arranged at other positions.

Further, the adherent cell culture vessel of this embodiment is preferably configured such that a plurality of intermediate culture walls are disposed between a first vessel wall and a second vessel wall, a culture chamber is disposed between the intermediate culture walls, and a flow passage communicating between the respective culture chambers is disposed.

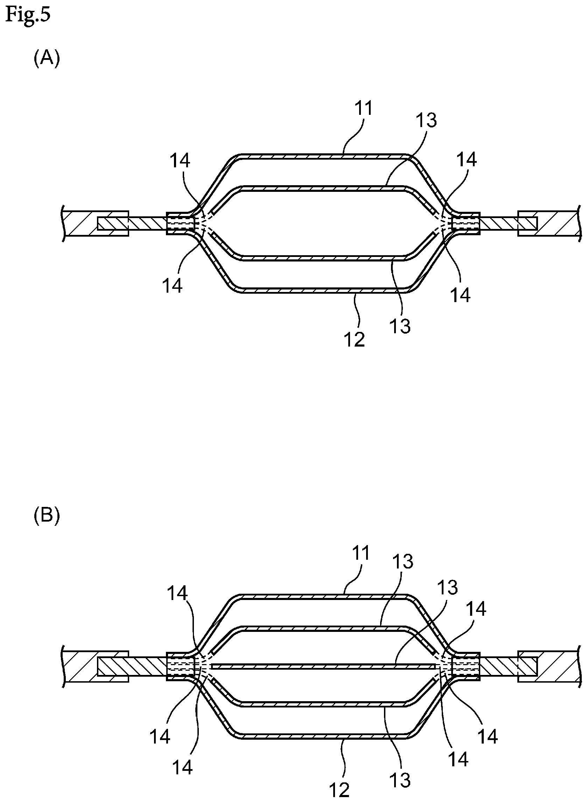

For example, it is also preferable that, as illustrated in Fig. 5A, two layers of the intermediate culture walls 13 are disposed between the first vessel wall 11 and the second vessel wall 12, and a culture chamber is each disposed between the first vessel wall 11 and the intermediate culture wall 13, between the two layers of the intermediate culture walls 13, and between the second vessel wall 12 and the intermediate culture wall 13, and the flow passages 14 communicating between the culture chambers are disposed.

When the adherent cell culture vessel is thus configured, as the culture portions in the adherent cell culture vessel, a total of six surfaces of the culture portion 111, the culture portion 121, and the four culture portions 131 can be used. Therefore, compared with a case where a double-sided culture is conducted using a culture vessel that does not include the intermediate culture wall 13, the culture area can be approximately tripled.

Since the adherent cell culture vessel is a bag-shaped vessel made of flexible packaging material even though the vessel is thus configured, the capacity in the vessel can be controlled depending on the amounts of a medium and a dissociation solution injected into the vessel, and dissociation and recovery of the cells can be easily performed without consuming the medium or the dissociation solution more than necessary.

Further, it is also preferable that, as illustrated in Fig. 5B, three layers of the intermediate culture walls 13 are disposed between the first vessel wall 11 and the second vessel wall 12, and a culture chamber is each disposed between the first vessel wall 11 and the intermediate culture wall 13, between the three layers of the intermediate culture walls 13, and between the second vessel wall 12 and the intermediate culture wall 13, and the flow passages 14 communicating between the culture chambers are disposed.

When the adherent cell culture vessel is thus configured, as the culture portions in the adherent cell culture vessel, a total of eight surfaces of the culture portion 111, the culture portion 121, and the six culture portions 131 can be used. Therefore, compared with a case where a double-sided culture is conducted using a culture vessel that does not include the intermediate culture wall 13, the culture area can be approximately quadrupled.

In the adherent cell culture vessel of this embodiment, four layers or more of the intermediate culture walls 13 can be disposed between the first vessel wall 11 and the second vessel wall 12, and by increasing the culture area accordingly, culture efficiency can be further improved.

Note that, while, in the adherent cell culture vessels in Fig. 5A and Fig. 5B, the thickness of the ports 2 is slightly thick compared with that in Fig. 1, this is due to a drawing reason and the thickness of the ports 2 may be identical.

In the adherent cell culture vessel of this embodiment, it is also possible not to use a part of the culture portion as a culture surface on which the adherent cells are cultured. This can be changed depending on whether or not surface treatment is performed on the culture portion. The surface treatment will be described later.

In the adherent cell culture vessel of this embodiment, as illustrated in Fig. 1, the first vessel wall 11 and the second vessel wall 12 preferably have bulging shapes that bulge outward in the peripheral edge regions of the culture chambers.

By thus configuring the first vessel wall 11 and the second vessel wall 12, the culture chambers can be formed more stably between the first vessel wall 11 and the intermediate culture wall 13 and between the second vessel wall 12 and the intermediate culture wall 13.

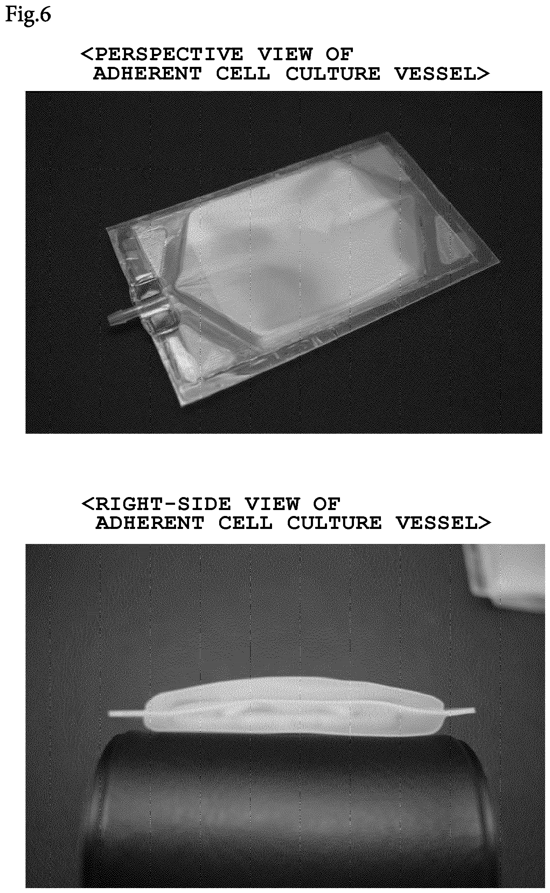

Fig. 6 illustrates photographs of the adherent cell culture vessel of this embodiment that is actually produced. In the drawing, the bulging shapes that bulge outward in the peripheral edge regions of the culture chambers of the adherent cell culture vessel are formed.

As illustrated in Fig. 5A and Fig. 5B, it is also preferable that the intermediate culture walls 13 have bulging shapes that bulge outward in the peripheral edge regions of the culture chambers.

By thus configuring the intermediate culture walls 13, the culture chambers can be formed more stably between the first vessel wall 11 and the intermediate culture wall 13, between the second vessel wall 12 and the intermediate culture wall 13, and between the plurality of intermediate culture walls 13.

In the adherent cell culture vessel of this embodiment, the thickness of the first vessel wall 11, the second vessel wall 12, and the intermediate culture wall 13 is preferably 1 mm or less.

When the adherent cell culture vessel of this embodiment is thus configured, the adherent cells can be easily dissociated by beating the culture vessel from the outside after a dissociation solution is used. Further, by setting the thickness of the first vessel wall 11 and the second vessel wall 12 to 1 mm or less, gas permeability in the adherent cell culture vessel can be enhanced.

In the adherent cell culture vessel of this embodiment, at least the whole or a part of the culture chamber side surface of the first vessel wall 11 or the first vessel wall side surface of the intermediate culture wall 13 and at least the whole or a part of the culture chamber side surface of the second vessel wall 12 or the second vessel wall side surface of the intermediate culture wall 13 are preferably formed by being provided with surface treatment for allowing the adherent cells to adhere to the surfaces.

By thus configuring the adherent cell culture vessel of this embodiment, the culture portions can be preferably formed in the adherent cell culture vessel.

As the surface treatment, for example, corona treatment or excimer treatment can be performed.

The adherent cell culture vessel of this embodiment preferably has a configuration in which a plurality of grooves composed of mountain-shaped portions and trough-shaped portions are disposed in the region of each of the surfaces of the culture portions of the first vessel wall 11, the second vessel wall 12, and the intermediate culture wall 13, on which the surface treatment is performed.

Furthermore, it is preferable that the groove has an approximately V-shaped cross section perpendicular to a direction in which the groove extends (hereinafter, the groove having this shape is referred to as a V-shaped groove in some cases) and an inclined angle of the side surfaces of the approximately V shape (hereinafter referred to as a V-shaped angle in some cases) in the groove is 80 degrees or less.

Here, when the V-shaped angle of the groove is 60 degrees, the surface area of the groove becomes twice the surface area of the corresponding region of a case where the groove is not disposed. Similarly, when the V-shaped angle is 65 degrees, the surface area of the groove becomes 2.37 times. When the V-shaped angle is 70 degrees, the surface area of the groove becomes 2.92 times. When the V-shaped angle is 75 degrees, the surface area of the groove becomes 3.86 times. When the V-shaped angle is 80 degrees, the surface area of the groove becomes 5.67 times.

Accordingly, from the aspect of the culture area, it is preferable that the V-shaped angle is larger. On the other hand, when the V-shaped angle becomes larger, machining performance deteriorates, for example, chipping occurring at the distal end portion of a machining mold, and the distal end of a machined object deforming. When the V-shaped angle is 83 degrees, the surface area of the groove becomes 8.21 times. However, it is difficult to make a machining mold with this V-shaped angle. Mostly, creating a machined object by making and using a machining mold having the V-shaped angle of 80 degrees is the limit.

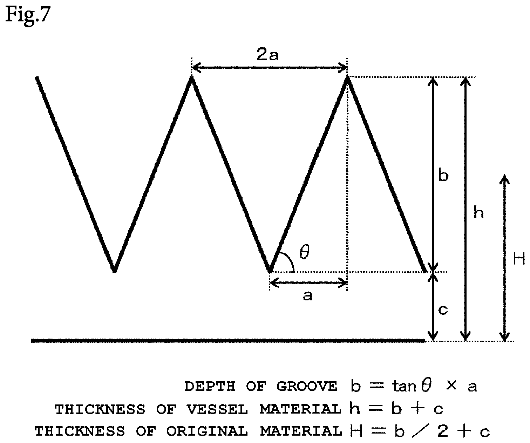

Next, the configuration of the V-shaped groove will be described with reference to Fig. 7.

In the drawing, an angle θ is the inclined angle (V-shaped angle) of the V-shaped groove and indicates an angle formed by a side surface of the V-shaped groove and a surface that is parallel to the other surface (vessel material lower surface) of a vessel material (film) constituting the first vessel wall 11, the second vessel wall 12, and the intermediate culture wall 13 and passes through the lowest point of the groove.

A distance a is a horizontal distance from the top end portion of a mountain-shaped portion to the center of the groove (which is equal to a horizontal distance from the lowest point of the groove to the center of the mountain-shaped portion). The distance (pitch) between the top end portions of mountain-shaped portions becomes 2a.

A distance ***b*** is the depth of the groove and indicates a vertical distance from the top end portion of the mountain-shaped portion to a plane that passes through the lowest point of the groove. That is, the depth of the groove is calculated by b = tanθ × a.

A distance ***c*** is the thickness of the bottom portion of the vessel material and indicates a distance in which the depth of the groove is subtracted from the thickness of the vessel material.

A distance ***h*** is the thickness of the vessel material and calculated by h = b + c = tanθ × a + c.

A distance H is the thickness of an original material used for production of the vessel material and calculated by H = b/2 + c = tanθ × a/2 + c.

The vessel material can be preferably produced by placing a molding die on a material to be worked and performing heat transfer. At this time, since the grooves are formed by pressing projecting portions of the molding die corresponding to the grooves of the vessel material against the material and the mountain-shaped portions are formed by pushing aside the material that has existed in the groove parts, the thickness of the vessel material becomes thicker than the thickness of the original material. The V-shaped groove is configured to extend in a vertical direction with respect to the paper surface of Fig. 7, and in the drawing, the relationship of a × (H - c) = a × b/2 is satisfied. In view of this, a thickness H of the original material is calculated by the above formula.

In the adherent cell culture vessel of this embodiment, it is preferable that the plurality of grooves are linearly disposed in parallel and the top end portions of the mountain-shaped portions are linear.

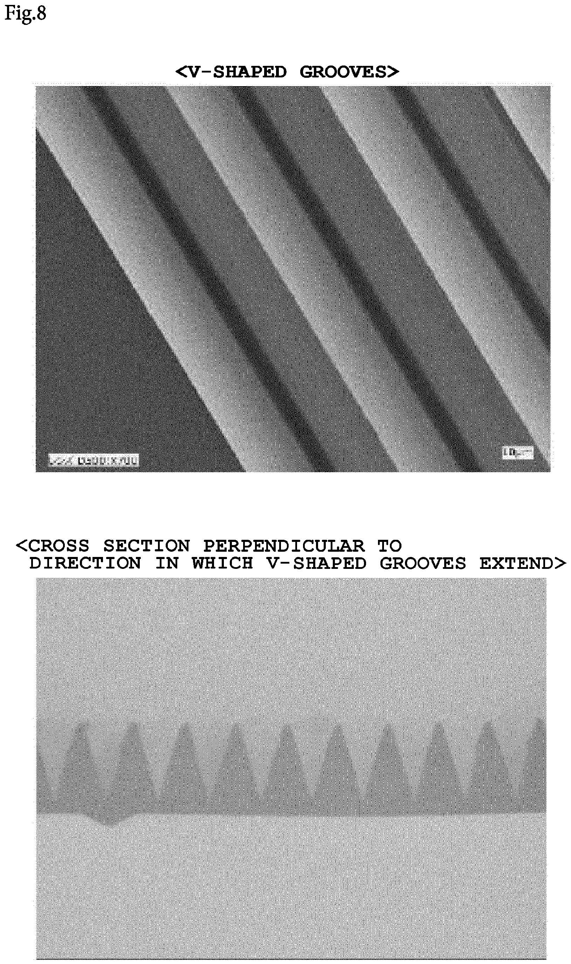

Specifically, as illustrated in the upper photograph (V-shaped grooves) of Fig. 8, it is preferable to configure to include the V-shaped grooves in a line shape. The cross section of the grooves is illustrated in the lower photograph (cross section perpendicular to a direction in which the V-shaped grooves extend) of Fig. 8.

When the adherent cell culture vessel of this embodiment is thus configured, the adherent cells are allowed to grow along the grooves in the line shape, and therefore the culture area can be effectively increased.

Further, the adherent cell culture vessel of this embodiment preferably has a configuration in which the plurality of grooves are disposed in which the grooves extend in direction nonparallel to one another on the surfaces facing one another.

When the adherent cell culture vessel of this embodiment is thus configured, the directions in which the grooves disposed on the surfaces facing one another extend can be shifted, and separation of the cells that have adhered by the grooves mutually overlapping can be avoided.

Further, for example, as illustrated in Fig. 1, the adherent cell culture vessel of this embodiment preferably has a configuration in which a plurality of ports 2 are disposed to be opposed in the peripheral edge regions of the culture chambers.

When the adherent cell culture vessel of this embodiment is thus configured, the medium and the dissociation solution can be efficiently injected into and discharged out of the adherent cell culture vessel.

The method for producing the adherent cell culture vessel of this embodiment is a method for producing a bag-shaped adherent cell culture vessel made of flexible packaging material, in which a plurality of grooves composed of mountain-shaped portions and trough-shaped portions are formed on three or more flexible packaging material films, the surfaces of the flexible packaging material films on which the grooves are formed are arranged in the culture vessel, the peripheral edge portions of the flexible packaging material films are heat-sealed with one or more injecting/ejecting ports interposed between the flexible packaging material films, vacuum forming, pressure forming, or vacuum pressure forming is performed to form culture chambers between the flexible packaging material films and include flow passages communicating between the respective culture chambers.

Specifically, for example, after surface treatment is performed on respective culture portions in the first vessel wall 11, the second vessel wall 12, and the intermediate culture wall 13, a plurality of V-shaped grooves are linearly formed in the culture portions.

Next, in the intermediate culture wall 13, cutouts (or holes) for forming the flow passages between the culture chambers are formed. As described above with reference to Fig. 4, when the intermediate culture wall 13 (13b) shorter than the first vessel wall 11 and the second vessel wall 12 is used, the cutouts (or holes) do not have to be formed.

Furthermore, seal portions at peripheral edges are heat-sealed with the intermediate culture wall 13 as well as the ports 2 interposed between the first vessel wall 11 and the second vessel wall 12, thereby making the adherent cell culture vessel into a bag.

At this time, the culture portion in which the plurality of V-shaped grooves are formed is arranged on at least one of the culture portions facing one another of the first vessel wall 11 and the intermediate culture wall 13. Further, the culture portion in which the plurality of V-shaped grooves are formed is arranged on at least one of the culture portions facing one another of the second vessel wall 12 and the intermediate culture wall 13.

Then, vacuum forming, pressure forming, or vacuum pressure forming is performed to form the first vessel wall 11 and the second vessel wall 12 so as to have bulging shapes.

At this time, air can be uniformly flowed in the respective culture chambers in the adherent cell culture vessel to preferably produce the adherent cell culture vessel of this embodiment including the flow passages 14 communicating between the respective culture chambers.

With the method for producing the adherent cell culture vessel of this embodiment, the adherent cell culture vessel including four or more surfaces of culture portions can be preferably produced relatively with ease.

Further, it is also preferable that the method for producing the bag-shaped adherent cell culture vessel of this embodiment is a method for producing a bag-shaped adherent cell culture vessel made of flexible packaging material, in which, bulging shapes that bulge outward in the peripheral edge regions of two flexible packaging material films are formed by vacuum forming, pressure forming, or vacuum pressure forming to configure a first vessel wall and a second vessel wall, bulging shapes that bulge outward in the peripheral edge regions of flexible packaging material films used other than for the intermediate culture wall arranged in the center, when a plurality of intermediate culture walls are disposed in the adherent cell culture vessel, are formed by vacuum forming, pressure forming, or vacuum pressure forming to configure the respective intermediate culture walls, the peripheral edge portions of the flexible packaging material films are heat-sealed with one or more injecting/ejecting ports and the intermediate culture walls interposed between the first vessel wall and the second vessel wall to form culture chambers between the flexible packaging material films and include flow passages communicating between the respective culture chambers.

Specifically, for example, prior to bag-making of an adherent cell culture vessel is performed, with respect to respective flexible packaging material films of the first vessel wall 11, the second vessel wall 12, and the intermediate culture walls 13 (excluding the center layer), bulging shapes that bulge outward in regions that become the peripheral edges of culture chambers are formed by vacuum forming, pressure forming, or vacuum pressure forming.

After surface treatment is performed on respective culture portions in the first vessel wall 11, the second vessel wall 12, and the intermediate culture walls 13, a plurality of V-shaped grooves are preferably linearly formed in the culture portions. Further, in the intermediate culture walls 13, cutouts (or holes) for forming flow passages between the culture chambers are formed. As described above with reference to Fig. 4, when the intermediate culture wall 13 (13b) shorter than the first vessel wall 11 and the second vessel wall 12 is used, the cutouts (or holes) do not have to be formed.

In the adherent cell culture vessel, in a case where a plurality of intermediate culture walls 13 are disposed, or in a case where both culture portions facing one another are planes, performing vacuum forming, pressure forming, or vacuum pressure forming after bag-making does not allow air to uniformly flow in the respective culture chambers in the adherent cell culture vessel in some cases.

Therefore, in these cases, it is preferable that, by heat-sealing seal portions at peripheral edges with the intermediate culture walls 13 and the ports 2 interposed between the first vessel wall 11 and the second vessel wall 12 after the bulging shapes are formed on the first vessel wall 11, the second vessel wall 12, and the intermediate culture walls 13, the adherent cell culture vessel including the flow passages communicating between the respective culture chambers is made into a bag.

With the method for producing the adherent cell culture vessel of this embodiment, even in a case where both culture portions facing one another of the first vessel wall 11, the second vessel wall 12, and the intermediate culture walls 13 are planes or in a case where a plurality of intermediate culture walls 13 are disposed, the adherent cell culture vessel can be preferably produced.

As described above, with the adherent cell culture vessel, and the method for producing the adherent cell culture vessel of this embodiment, since four or more surfaces of culture portions are disposed in a bag-shaped culture vessel made of flexible packaging material, the surface area of the culture portions can be increased and dissociation and recovery of cells can be easily performed without consuming a medium or a dissociation solution more than necessary.

That is, since the capacity in the vessel can be controlled depending on the amounts of the medium and the dissociation solution injected into the vessel, unnecessary consumption can be reduced without consuming the medium or the dissociation solution more than necessary. Further, by applying a force from the outside of the adherent cell culture vessel or by bringing the culture portions into contact with one another after the dissociation solution is made to act, the cells can be easily recovered.

Furthermore, by including a plurality of grooves composed of mountain-shaped portions and trough-shaped portions in the culture portions, the surface area of the culture portions can be further increased.

The present invention is not limited to the above-mentioned embodiment, and it is needless to say that various changes are possible within the scope of the present invention. For example, the grooves in the culture portions of the adherent cell culture vessel can be appropriately changed by, for example, forming the grooves not linearly but in a curved line. Further, a cell culture vessel applicable to culture of floating cells can be configured without performing surface treatment on the culture portions.

### INDUSTRIAL APPLICABILITY

The present invention can be preferably used, for example, when adherent cells are efficiently made in a large amount.

The documents described in the specification and the specifications of Japanese applications on the basis of which the present application claims Paris convention priority are incorporated herein by reference in its entirety.

### DESCRIPTION OF REFERENCE SIGNS

- 1: Vessel body portion
- 11: First vessel wall
- 111: Culture portion
- 112: Seal portion
- 12: Second vessel wall
- 121: Culture portion
- 122: Seal portion
- 13, 13a, 13b: Intermediate culture wall
- 131, 131a, 131b: Culture portion
- 132, 132a, 132b: Seal portion
- 14, 14a: Flow passage
- 2: Port
- 3: Tube

## Claims

1. A bag-shaped adherent cell culture vessel made of flexible packaging material, comprising:
a vessel body portion having a first vessel wall and a second vessel wall; and
one or more injecting/ejecting ports, wherein
the vessel body portion has an intermediate culture wall between the first vessel wall and the second vessel wall, and
a culture chamber is disposed in each of between the first vessel wall and the intermediate culture wall and between the second vessel wall and the intermediate culture wall, and a flow passage communicating between the respective culture chambers is disposed.

2. The adherent cell culture vessel according to claim 1, wherein
a plurality of the intermediate culture walls are disposed between the first vessel wall and the second vessel wall, a culture chamber is disposed between the intermediate culture walls, and a flow passage communicating between the respective culture chambers is disposed.

3. The adherent cell culture vessel according to claim 1 or 2, wherein
the flow passage is a hole or a cutout passing through the intermediate culture wall.

4. The adherent cell culture vessel according to any of claims 1 to 3, wherein
the first vessel wall and the second vessel wall have a bulging shape that bulges outward in a peripheral edge region of a culture chamber.

5. The adherent cell culture vessel according to any of claims 1 to 4, wherein
the intermediate culture wall has a bulging shape that bulges outward in a peripheral edge region of a culture chamber.

6. The adherent cell culture vessel according to any of claims 1 to 5, wherein
a thickness of the first vessel wall, the second vessel wall, and the intermediate culture wall is 1 mm or less.

7. The adherent cell culture vessel according to any of claims 1 to 6, wherein
at least a whole or a part of a culture chamber side surface of the first vessel wall or a first vessel wall side surface of the intermediate culture wall and at least a whole or a part of a culture chamber side surface of the second vessel wall or a second vessel wall side surface of the intermediate culture wall are formed by being provided with surface treatment for allowing an adherent cell to adhere to surfaces thereof.

8. The adherent cell culture vessel according to claim 7, wherein
a plurality of grooves composed of mountain-shaped portions and trough-shaped portions are disposed in a region of each of the surfaces on which surface treatment is performed.

9. The adherent cell culture vessel according to claim 8, wherein
the grooves have an approximately V-shaped cross section perpendicular to a direction in which the groove extends, and an inclined angle of side surfaces of the approximately V shape in the groove is 80 degrees or less.

10. The adherent cell culture vessel according to claim 8 or 9, wherein
the grooves are linearly disposed in parallel, and a top end portion of the mountain-shaped portion is linear.

11. The adherent cell culture vessel according to claim 10, wherein
the grooves are disposed in which the grooves extend in directions nonparallel to one another on the surfaces facing one another.

12. The adherent cell culture vessel according to any of claims 1 to 11, wherein
a plurality of the injecting/ejecting ports are disposed to be opposed in a peripheral edge region of a culture chamber.

13. A bag-shaped cell culture vessel made of flexible packaging material, comprising:
a vessel body portion having a first vessel wall and a second vessel wall; and
one or more injecting/ejecting ports, wherein
the vessel body portion has an intermediate culture wall between the first vessel wall and the second vessel wall, and
a culture chamber is disposed in each of between the first vessel wall and the intermediate culture wall and between the second vessel wall and the intermediate culture wall, and a flow passage communicating between the respective culture chambers is disposed.

14. A method for producing a bag-shaped adherent cell culture vessel made of flexible packaging material, the method comprising:
forming a plurality of grooves composed of mountain-shaped portions and trough-shaped portions on three or more flexible packaging material films;
arranging a surface of the flexible packaging material films on which the grooves are formed in the culture vessel;
heat-sealing a peripheral edge portion of the flexible packaging material films with one or more injecting/ejecting ports interposed between the flexible packaging material films; and
performing vacuum forming, pressure forming, or vacuum pressure forming to form a culture chamber between the flexible packaging material films and a flow passage communicating between the respective culture chambers.

15. A method for producing a bag-shaped adherent cell culture vessel made of flexible packaging material, the method comprising:
forming a bulging shape that bulges outward in a peripheral edge region of two flexible packaging material films by vacuum forming, pressure forming, or vacuum pressure forming to form a first vessel wall and a second vessel wall;
forming a bulging shape that bulges outward in a peripheral edge region of flexible packaging material films used other than for an intermediate culture wall arranged in a center, when a plurality of intermediate culture walls are disposed in the adherent cell culture vessel, by vacuum forming, pressure forming, or vacuum pressure forming to form respective intermediate culture walls; and
heat-sealing a peripheral edge portion of the flexible packaging material films with one or more injecting/ejecting ports and the intermediate culture walls interposed between the first vessel wall and the second vessel wall to form a culture chamber between the flexible packaging material films and a flow passage communicating between the respective culture chambers.
